# EUROPÄISCHE PATENTSCHRIFT

(11) **EP 0 397 977 B1**
(45) Veröffentlichungstag und Bekanntmachung des Hinweises auf die Patenterteilung: **21.07.1993**
(21) Anmeldenummer: 90104571.6
(22) Anmeldetag: 10.03.1990
(51) Int. Cl.: A61M 5/31

(54) **Spritze für medizinische Zwecke**
Syringe for medical use
Seringue à usage médical

(30) Priorität: 17.05.1989 DE 3916101
(43) Veröffentlichungstag der Anmeldung: 22.11.1990
(73) Patentinhaber: Arzneimittel GmbH Apotheker Vetter & Co. Ravensburg, D-88212 Ravensburg (DE)
(72) Erfinder: Vetter, Helmut, D-7980 Ravensburg (DE); Geprägs, Peter, D-7987 Weingarten (DE)
(74) Vertreter: Fay, Hermann, Dipl.-Phys. Dr.

(56) Entgegenhaltungen:
- EP-A- 0 112 574
- EP-A- 0 207 544
- US-A- 2 679 246
- US-A- 3 659 749

## Beschreibung

Die Erfindung betrifft eine Spritze für medizinische Zwecke, mit einem Spritzenzylinder und zumindest einem darin mittels einer Kolbenstange verschiebbaren Spritzenkolben, sowie mit einem kanülenseitigen Verschlußteil für den Spritzenzylinder und einer Fingerauflage am dem Verschlußteil entgegengesetzten Ende des Spritzenzylinders, wobei der Spritzenzylinder als zylindrisches Rohr ausgebildet ist, das in axialer Richtung stirnseitig einer flachzylindrischen Dichtscheibe aus vergleichsweise weich eingestelltem gummielastischem Werkstoff anliegt, die in einen Aufnahmeflansch des Verschlußteils eingelegt ist und den Spritzenzylinder gegen das Verschlußteil abdichtet.

Eine derartige Spritze ist aus der US-A-2 679 246 bekannt. Bei dieser Spritze ist das kanülenseitige Ende des Spritzenzylinders mit einem Außengewinde versehen, auf das das Verschlußteil mit einem entsprechenden Innengewinde aufgeschraubt ist.

Ein derartiger Aufbau erschwert die automatisierte Montage und Konfektionierung der Spritzen, da die zur Verschraubung notwendige Drehbewegung an jedem einzelnen Spritzenzylinder aufgebracht werden muß, was insbes. die gleichzeitige Verarbeitung einer Vielzahl von Spritzen erschwert.

Die EP-A-0 112 574 beschreibt eine Spritze mit einem Verschlußteil, die am kanülenseitigen Ende des Spritzenzylinders mit einem Außenbund versehen ist, der das Verschlußteil trägt. Das Verschlußteil weist auf der der Kanüle zugewandten Seite eine axial verlaufende Bohrung auf, die in eine radial ausgerichtete Bohrung mündet. Das Verschlußteil weist zwei Rastsitze für den Außenbund am Spritzenzylinder auf, die so angeordnet sind, daß je nach Rastsitz die Mündungen der radial verlaufenden Bohrung durch die innere Mantelfläche des Spritzenzylinders abgedichtet bzw. offen sind.

Der Erfindung liegt die Aufgabe zugrunde, eine Spritze der eingangs genannten Art so auszubilden, daß sie aus einfach herzustellenden Bestandteilen auf fertigungstechnisch einfache Weise vollständig zusammengesetzt und fertig konfektioniert werden kann, wobei zugleich eine wirksame Abdichtung zwischen dem Spritzenzylinder und dem Verschlußteil erreicht wird.

Diese Aufgabe wird nach der Erfindung dadurch gelöst, daß der Spritzenzylinder zumindest an seinem kanülenseitigen Ende einen Ringbund aufweist, der einen radial nach außen vorstehenden Rastvorsprung für das Verschlußteil bildet und daß das Verschlußteil mit einer lösbar aufgesteckten Konuskappe (Tip-Cap) verschlossen ist, die mit einem axial ausgerichteten Zapfen versehen ist, der in die zur Aufnahme der Kanüle eingerichtete Durchgangsbohrung des Verschlußteils vorsteht, wobei die Dichtscheibe mit einer zentralen Öffnung versehen und das freie Ende des Zapfens dem Innenrand der Öffnung dichtend anliegt.

Der durch die Erfindung erreichte Vorteil besteht im wesentlichen darin, daß einfach herzustellende Bestandteile, nämlich ein rein zylindrisches Rohr ohne jegliche Querschnittsänderung für den Spritzenzylinder verwendet werden und das Verschlußteil einfach aufgebaut sein kann. Zur Fertigstellung und Konfektionierung der Spritzen muß das Verschlußteil lediglich in axialer Richtung auf den Spritzenzylinder aufgesetzt werden, wodurch jegliche Dreh- oder Schraubbewegung entfällt. Dies ermöglicht es, eine Vielzahl von untereinander parallel ausgerichteten Spritzen gleichzeitig durch einen einzigen Bewegungshub fertigzustellen, was insbes. für nur einmal zu verwendende und daher möglichst preisgünstig herzustellende Einwegspritzen von Bedeutung ist.

In bevorzugter Ausführungsform der Erfindung kann das Verschlußteil mit einem Anschlußhals zum Ansatz einer mit einer Nadelmuffe versehenen Kanüle versehen sein. Die Nadel wird dann nach Entfernen der Konuskappe (Tip-Cap) auf den Anschlußhals aufgesetzt.

Ist die Spritze als vorgefüllte Fertigspritze ausgebildet, die zusätzlich mit einem Mittelstopfen versehen ist, wobei die Kammer zwischen dem Spritzenkolben und dem Mittelstopfen zur Aufnahme eines Lösungsmittels dient und die kanülenseitige Kammer den Wirkstoff in ungelöster, lyophilisierter Form enthält und wobei der Spritzenzylinder einen Bypass zum Übertritt des Lösungsmittels in die kanülenseitige Kammer aufweist, so ist im Rahmen der Erfindung weiter vorgesehen, daß das Verschlußteil zumindest zwei Raststellungen auf dem Spritzenzylinder aufweist und mit in den Aufnahmeflansch mündenden, radial verlaufenden Durchgangsbohrungen versehen ist, wobei die Durchgangsbohrungen in der in Aufsteckrichtung des Verschlußteils auf den Spritzenzylinder ersten Raststellung mit der kanülenseitigen Kammer in Verbindung stehen und in der zweiten Raststellung, in der der Spritzenzylinder stirnseitig der Dichtscheibe anliegt, durch die Außenmantelfläche des Spritzenzylinders überdeckt sind. So lange sich hierbei das Verschlußteil in seiner ersten Raststellung befindet, besteht über die Durchgangsbohrungen eine Verbindung der kanülenseitigen Kammer nach außen, so daß der in diese Kammer in gelöster Form eingebrachte Wirkstoff über die Durchgangsbohrung lyophilisiert werden kann. Anschließend wird diese Kammer durch einfaches weiteres Aufdrücken des Verschlußteils in die zweite Raststellung steril abgeschlossen.

Bei einer solchen Spritze kann die Kolbenstange auf der zum Spritzenkolben weisenden Seite mit einem Außengewinde und die Fingerauflage mit einem entsprechenden Innengewinde versehen sein, während die dem Spritzenkolben abgewandte Seite der Kolbenstange einen deren freie Verschiebbarkeit ermöglichenden geringeren Durchmesser besitzt. Der Spritzenkolben läßt sich daher aus seiner Ausgangsstellung zum kanülenseitigen Ende hin nur durch eine Drehbewegung des Spritzenkolbens verstellen, wodurch sich selbsttätig eine Begrenzung der Verstellgeschwindigkeit des Kolbens ergibt. Hierdurch ist sicher gestellt, daß das Lösungsmittel in die den Wirkstoff enthaltende vordere Kammer langsam übergeführt wird. Erst wenn das Gewinde der Kolbenstange aus der Fingerauflage freikommt, läßt sich der Spritzenkolben allein durch Druck auf die Kolbenstange zur Applikation des dann gelösten Wirkstoffes verstellen.

Schließlich kann im Rahmen der Erfindung noch vorgesehen sein, daß auf das Verschlußteil eine Sicherungskappe aufgeschoben ist, die das Verschlußteil formschlüssig umgreift, wobei die Sicherungskappe an ihrer der Fingerauflage zugewandten Seite einen Klemmring trägt, der über einen dünnen Trennsteg an der Sicherungskappe angeschlossen ist und in vollständig aufgeschobenem Zustand unlösbar mit dem Verschlußteil verbunden ist und wobei die Sicherungskappe innenseitig einen axial ausgerichteten Zapfen trägt, der die Kanülenöffnung des Verschlußteils schließt. Die Unversehrtheit der Sicherungskappe stellt dabei sicher, daß der Inhalt der Spritze sich noch in seinem ursprünglichen, sterilen Zustand befindet.

Im folgenden wird die Erfindung an in der Zeichnung dargestellten Ausführungsbeispielen näher erläutert; es zeigen:
- Fig. 1: den Gegenstand nach der Erfindung in einer ersten Ausführungsform mit aufgesetzter Konuskappe, (Tip-Cap)
- Fig. 2: den Gegenstand nach Fig. 1, jedoch mit angesetzter Kanüle,
- Fig. 3: eine zur Lyophilisierung geeignete Ausführungsvariante in Lyophilisationsstellung,
- Fig. 4: den Gegenstand nach Fig. 3, jedoch nach erfolgter Lyophilisierung mit vollständig aufgeschobenem Verschlußteil,
- Fig. 5: eine weitere Ausgestaltung einer Spritze nach den Fig. 3 und 4,
- Fig. 6: eine Spritze in der Fig. 1 entsprechender Gestaltung, jedoch mit einem Originalitätsverschluß.

Die in der Zeichnung dargestellte Spritze für medizinische Zwecke besteht aus einem Spritzenzylinder 1 und zumindest einem darin mittels einer Kolbenstange 2 verschiebbaren Spritzenkolben 3. An seinem einen Ende ist der Spritzenzylinder 1 mit einem Verschlußteil 4 versehen, das zugleich zur Aufnahme der Kanüle 5 dient, während am entgegengesetzten Ende eine übliche Fingerauflage 6 angeordnet ist.

Der Spritzenzylinder 1 ist im einzelnen als zylindrisches Rohr ausgebildet, daß an seinem kanülenseitigen Ende einen Ringbund 1.1 aufweist. Dieser Ringbund 1.1 bildet einen radial nach außen vorstehenden Rastvorsprung für das Verschlußteil 4 und liegt in axialer Richtung stirnseitig einer flachzylindrischen Dichtscheibe 7 aus vergleichsweise weich eingestelltem gummielastischem Werkstoff an. Diese Dichtscheibe 7 ist in einen Aufnahmeflansch 4.1 des Verschlußteils 4 eingelegt und dichtet den Spritzenzylinder 1 gegen das Verschlußteil 4 ab.

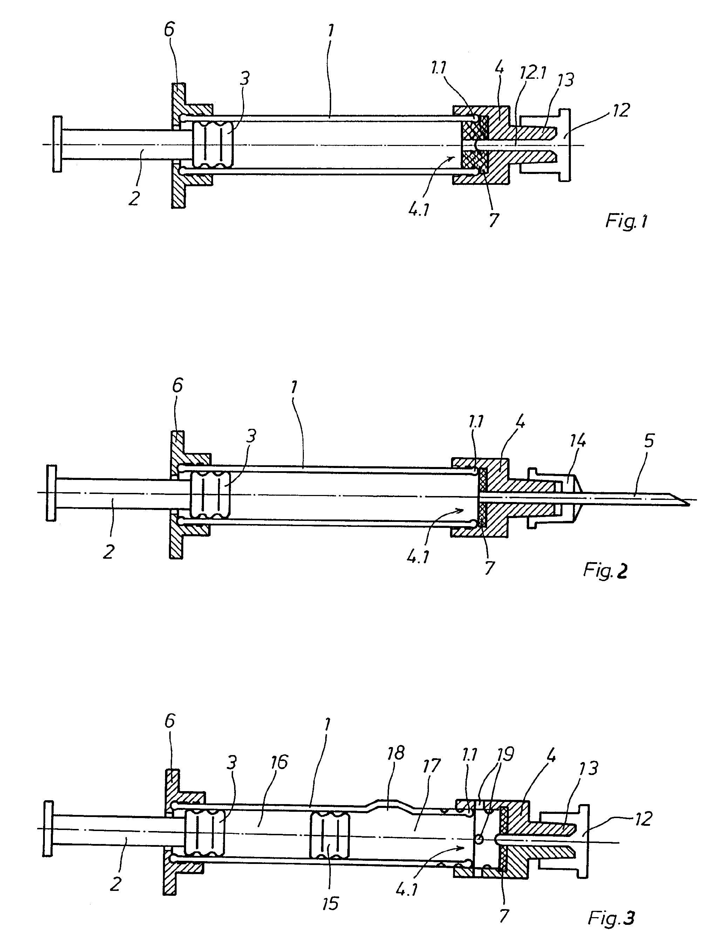

Die Spritze ist zunächst mit einer lösbar auf das Verschlußteil 4 aufgesteckten Konuskappe (Tip-Cap) 12 zu verschlossen. Dazu ist die Konuskappe (Tip-Cap) 12 mit einem axial ausgerichteten Zapfen 12.1 versehen, der in die zur Aufnahme der Kanüle eingerichtete Durchgangsbohrung des Verschlußteils 4 vorsteht. Die Dichtscheibe 7 ist mit einer zentralen Öffnung 10 versehen, so daß sich der Zapfen 12.1 durch die Dichtscheibe 7 hindurch erstrecken und sich dabei mit seinem freien Ende dem Innenrand der Öffnung 10 dichtend anlegen kann. Das Verschlußteil 4 ist mit einem Anschlußhals 13 zum Ansatz einer die Nadelmuffe 14 tragenden Kanüle 5 versehen. In Fig. 2 ist eine solche Spritze mit aufgesetzter Nadel 5 dargestellt.

Die Fig. 3 bis 5 zeigen eine sogenannte Doppelkammerspritze, die zusätzlich mit einem Mittelstopfen 15 versehen ist, wobei die Kammer 16 zwischen dem Spritzenkolben 3 und dem Mittelstopfen 15 zur Aufnahme eines Lösungsmittels dient. Die kanülenseitige Kammer 17 enthält den Wirkstoff in ungelöster und in der Regel lyophilisierter Form. Ferner weist der Spritzenzylinder 1 einen Bypass 18 auf, der den Übertritt des Lösungsmittels aus der hinteren 16 in die kanülenseitige Kammer 17 ermöglicht. Solche Spritzen werden als sogenannte Fertigspritzen vorgefüllt in den Handel gebracht, wobei die Lyophilisierung unmittelbar in der Spritze erfolgt. Dazu ist das Verschlußteil 4 mit in den Aufnahmeflansch 4.1 mündenden, radial verlaufenden Durchgangsbohrungen 19 versehen, wobei das Verschlußteil 4 zumindest zwei Raststellungen auf den Spritzenzylinder 1 aufweist. In der in Aufsteckrichtung des Verschlußteils 4 auf den Spritzenzylinder 1 ersten Raststellung stehen die Durchgangsbohrungen 19 mit der kanülenseitigen Kammer 17 in Verbindung, so daß das bei der Lyphilisierung frei werdende Lösungsmittel über die Durchgangsbohrungen 19 austreten kann. Nach Abschluß der Lyophilisierung wird das Verschlußteil 4 in die zweite Raststellung gebracht, in der der Spritzenzylinder 1 stirnseitig der Dichtscheibe 7 anliegt und die Durchgangsbohrungen 19 durch die Außenmantelfläche des Spritzenzylinders 1 überdeckt sind.

Um eine kontinuierliche Überfüllung des Lösungsmittels aus der hinteren Kammer 16 in die kanülenseitige Kammer 17 zu gewährleisten, ist die Kolbenstange 2 auf der zum Spritzenkolben 3 weisenden Seite mit einem Außengewinde 2.1 und die Fingerauflage 6 mit einem entsprechenden Innengewinde versehen. Dadurch läßt sich der Spritzenkolben 3 nur durch Drehen der Kolbenstange 2 vorwärts bewegen, bis dessen Gewinde 2.1 aus der Fingerauflage 6 freikommt. Die dem Spritzenkolben 3 abgewandte Seite der Kolbenstange 2 besitzt dagegen einen deren freie Verschiebbarkeit ermöglichenden geringeren Durchmesser, so daß anschließend die Applikation des dann in gelöster Form vorliegenden Wirkstoffs in gewohnter Weise erfolgen kann.

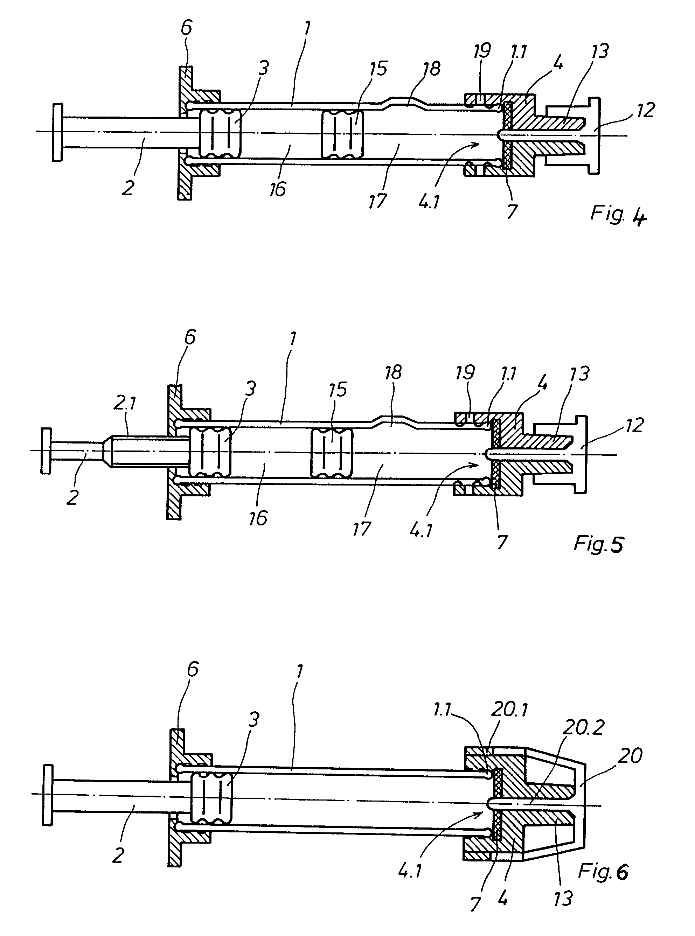

Um sicher zu stellen, daß eine insbesondere vorgefüllte Fertigspritze sich in ihrem original verschlossenen Zustand befindet, kann auf das Verschlußteil 4 eine Sicherungskappe 20 aufgeschoben sein, wie sich dies aus der Fig. 6 ergibt. Diese Sicherungskappe 20 umgreift das Verschlußteil 4 formschlüssig, wobei die Sicherungskappe 20 an ihrer der Fingerauflage 6 zugewandten Seite einen Klemmring 20.1 trägt. Dieser Klemmring 20.1 ist über einen in der Zeichnung nicht näher dargestellten dünnen Trennsteg an der Sicherungskappe 20 angeschlossen und in vollständig aufgeschobenem Zustand auf dem Verschlußteil 4 unlösbar mit diesem verbunden. Somit läßt sich die Spritze kanülenseitig nur durch Entfernen der Sicherungskappe 20 an dem als Sollbruchstelle ausgebildeten Trennsteg öffnen, jedoch nicht wieder verschließen. Dabei kann die Sicherungskappe innenseitig einen axial ausgerichteten Zapfen 20.1 tragen, der die Kanülenöffnung des Verschlußteils 4 schließt, so daß die Sicherungskappe 20 gleichzeitig als Tip-Cap wirkt.

## Patentansprüche

1. Spritze für medizinische Zwecke, mit einem Spritzenzylinder (1) und zumindest einem darin mittels einer Kolbenstange (2) verschiebbaren Spritzenkolben (3), sowie mit einem kanülenseitigen Verschlußteil (4) für den Spritzenzylinder (1) und einer Fingerauflage (6) am dem Verschlußteil entgegengesetzten Ende des Spritzenzylinders (1), wobei der Spritzenzylinder (1) als zylindrisches Rohr ausgebildet ist, das in axialer Richtung stirnseitig einer flachzylindrischen Dichtscheibe (7) aus vergleichsweise weich eingestelltem gummielastischem Werkstoff anliegt, die in einen Aufnahmeflansch (4.1) des Verschlußteils (4) eingelegt ist und den Spritzenzylinder (1) gegen das Verschlußteil (4) abdichtet, dadurch gekennzeichnet, daß der Spritzenzylinder (1) zumindest an seinem kanülenseitigen Ende einen Ringbund (1.1) aufweist, der einen radial nach außen vorstehenden Rastvorsprung für das Verschlußteil (4) bildet und daß das Verschlußteil (4) mit einer lösbar aufgesteckten Konuskappe (Tip-Cap) (12) verschlossen ist, die mit einem axial ausgerichteten Zapfen (12.1) versehen ist, der in die zur Aufnahme der Kanüle (5) eingerichtete Durchgangsbohrung des Verschlußteils (4) vorsteht, wobei die Dichtscheibe (7) mit einer zentralen Öffnung (10) versehen und das freie Ende des Zapfens (12.1) dem Innenrand der Öffnung (10) dichtend anliegt.

2. Spritze nach Anspruch 1, dadurch gekennzeichnet, daß das Verschlußteil (4) mit einem Anschlußhals (13) zum Ansatz einer mit einer Nadelmuffe (14) versehenen Kanüle (5) versehen ist.

3. Spritze nach Anspruch 1 als vorgefüllte Fertigspritze, die zusätzlich mit einem Mittelstopfen (15) versehen ist, wobei die Kammer (16) zwischen dem Spritzenkolben (3) und dem Mittelstopfen (15) zur Aufnahme eines Lösungsmittels dient und die kanülenseitige Kammer (17) den Wirkstoff in ungelöster lyophilisierter Form enthält und wobei der Spritzenzylinder (1) einen Bypass (18) zum Übertritt des Lösungsmittels in die kanülenseitige Kammer (17) aufweist, dadurch gekennzeichnet, daß das Verschlußteil (4) zumindest zwei Raststellungen auf dem Spritzenzylinder (1) aufweist und mit in den Aufnahmeflansch (4.1) mündenden, radial verlaufenden Durchgangsbohrungen (19) versehen ist, wobei die Durchgangsbohrungen (19) in der in Aufsteckrichtung des Verschlußteils (4) auf den Spritzenzylinder (1) ersten Raststellung mit der kanülenseitigen Kammer (17) in Verbindung stehen und in der zweiten Raststellung, in der der Spritzenzylinder (1) stirnseitig der Dichtscheibe (7) anliegt, durch die Außenmantelfläche des Spritzenzylinders (1) überdeckt sind.

4. Spritze nach Anspruch 3, dadurch gekennzeichnet, daß die Kolbenstange (2) auf der zum Spritzenkolben (3) weisenden Seite mit einem Außengewinde (2.1) und die Fingerauflage (6) mit einem entsprechenden Innengewinde versehen ist, während die dem Spritzenkolben (3) abgewandte Seite der Kolbenstange (2) einen deren freie Verschiebbarkeit ermöglichenden geringeren Durchmesser besitzt.

5. Spritze nach einem der Ansprüche 1 bis 4, dadurch gekennzeichnet, daß auf das Verschlußteil (4) eine Sicherungskappe (20) aufgeschoben ist, die das Verschlußteil (4) formschlüssig umgreift, wobei die Sicherungskappe (20) an ihrer der Fingerauflage (6) zugewandten Seite einen Klemmring (2.1) trägt, der über einen dünnen Trennsteg an der Sicherungskappe (20) angeschlossen ist und in vollständig aufgeschobenem Zustand unlösbar mit dem Verschlußteil (4) verbunden ist und wobei die Sicherungskappe (20) innenseitig einen axial ausgerichteten Zapfen (20.2) trägt, der die Kanülenöffnung des Verschlußteils (4) schließt.

## Claims

1. A syringe for medical purposes comprising a syringe cylinder (1) and at least one syringe plunger (3) which is displaceable therein by means of a plunger rod (2), and a closure portion (4) for the syringe cylinder (1) at the needle end and a finger support (6) at the end of the syringe cylinder (1) which is opposite the closure portion, wherein the syringe cylinder (1) is in the form of a cylindrical tube which bears at the end in the axial direction against a sealing disc (7) of a flat cylindrical configuration and of resilient material which is made comparatively soft, which sealing disc is inserted into a receiving flange (4.1) of the closure portion (4) and seals the syringe cylinder (1) relative to the closure portion (4), characterised in that at least at its needle end the syringe cylinder (1) has an annular collar (1.1) which forms a radially outwardly projecting retaining projection for the closure portion (4) and that the closure portion (4) is closed by a cone cap (tip-cap) (12) which is releasably fitted thereon and which is provided with an axially directed pin (12.1) which projects into the through bore in the closure portion (4), which is adapted to receive the needle (5), wherein the sealing disc (7) is provided with a central opening (10) and the free end of the pin (12.1) bears sealingly against the inside edge of the opening (10).

2. A syringe according to claim 1 characterised in that the closure portion (4) is provided with a connecting neck (13) for attachment of a needle (5) provided with a needle sleeve portion (14).

3. A syringe according to claim 1 in the form of a pre-filled ready-for-use syringe which is additionally provided with a central plug (15), wherein the chamber (16) between the syringe plunger (3) and the central plug (15) serves to accomodate a solvent and the chamber (17) at the needle end contains the active substance in undissolved lyophilised form and wherein the syringe cylinder (1) has a by-pass (18) for the solvent to be transferred into the chamber (17) at the needle end, characterised in that the closure portion (4) has at least two retaining positions on the syringe cylinder (1) and is provided with radially extending through bores (19) which open into the receiving flange (4.1), wherein the through bores (19) communicate with the chamber (17) at the needle end in the retaining position which is the first position in the direction of fitting the closure portion (4) on to the syringe cylinder (1) and are covered by the outside peripheral surface of the syringe cylinder (1) in the second retaining position in which the syringe cylinder (1) bears at the end against the sealing disc (7).

4. A syringe according to claim 3 characterised in that on the side which is towards the syringe plunger (3) the plunger rod (2) is provided with an external screwthread (2.1) and the finger support (6) is provided with a corresponding internal screwthread while the side of the plunger rod (2), which is remote from the syringe plunger (3), is of a smaller diameter which permits free displaceability thereof.

5. A syringe according to one of claims 1 to 4 characterised in that pushed on to the closure portion (4) is a safety cap (20) which positively lockingly embraces the closure portion (4), wherein at its side which is towards the finger support (6) the safety cap (20) carries a clamping ring (20.1) which is connected to the safety cap (20) by way of a thin separating limb and which in the fully pushed-on condition is non-releasably connected to the closure portion (4), and wherein the safety cap (20) carries on its inside an axially directed pin (20.2) which closes the needle opening in the closure portion (4).

## Revendications

1. Seringue à usage médical, comportant un cylindre (1) et au moins un piston (3) déplaçable à l'intérieur du cylindre au moyen d'une tige de piston (2), ainsi qu'un élément de fermeture (4) situé du côté de la canule, pour le cylindre (1) de la seringue, et un appui-doigt (6), situé sur l'extrémité du cylindre (1) de la seringue, tournée à l'opposé de l'élément de fermeture, et dans laquelle le cylindre (1) de la seringue est réalisé sous la forme d'un tube cylindrique, qui s'applique frontalement, dans la direction axiale, contre un disque d'étanchéité (7) en forme de cylindre plat, réalisé en un matériau présentant l'élasticité du caoutchouc et réglé de manière à être comparativement mou et qui est inséré dans une bride de logement (4.1) de l'élément de fermeture (4) et établit l'étanchéité entre le cylindre (1) de la seringue et l'élément de fermeture (4), caractérisée en ce que le cylindre (1) de la seringue possède, au moins sur son extrémité tournée du côté de la canule, un collet annulaire (1.1), qui forme une partie saillante d'encliquetage, qui fait saillie radialement vers l'extérieur, pour l'élément de fermeture (4), et que l'élément de fermeture (4) est fermé par un capuchon conique emmanché de façon amovible (Tip-Cap) (12), qui comporte une tige axiale (12.1) qui pénètre dans le perçage traversant de l'élément de fermeture (4), qui est agencé de manière à loger la canule (5), le disque d'étanchéité (7) possédant une ouverture centrale (10), tandis que l'extrémité libre de la tige (12.1) s'applique d'une manière étanche contre le bord intérieur de l'ouverture (10).

2. Seringue selon la revendication 1, caractérisée en ce que l'élément de fermeture (4) comporte un col de raccordement (13) permettant le montage d'une canule (5) équipée d'un manchon d'aiguille (14).

3. Seringue selon la revendication 1, réalisée sous la forme d'une seringue prête à utilisation, préalablement remplie, qui est équipée en outre d'un bouchon médian (15), et dans laquelle la chambre (16) située entre le piston (3) de la seringue et le bouchon médian (15) sert à loger un solvant, et la chambre (17), située du côté de la canule, contient la substance active sous forme lyophilisée non dissoute, et dans laquelle le cylindre (1) de la seringue comporte un by-pass (18) pour le transfert du solvant dans la chambre (17) située du côté de la canule, caractérisée en ce que l'élément de fermeture (4) possède au moins deux positions d'encliquetage sur le cylindre (1) de la seringue et est pourvu de perçages traversants radiaux (19), qui débouchent dans la bride de logement (4.1), les perçages traversants (19) étant en liaison avec la chambre (17) située du côté de la canule, lorsque l'élément de fermeture (4) est dans sa première position dans sa direction d'emmanchement sur le cylindre (1) de la seringue, et sont recouverts par la surface enveloppe extérieure du cylindre (1) de la seringue, lorsque l'élément de fermeture est dans la seconde position d'encliquetage, dans laquelle le cylindre (1) de la seringue s'applique frontalement contre le disque d'étanchéité (7).

4. Seringue selon la revendication 3, caractérisée en ce que la tige de piston (2) comporte, sur le côté tourné vers le piston (3) de la seringue, un filetage extérieur (2.1), et l'appui-doigt (6) comporte un taraudage correspondant, tandis que le côté de la tige de piston (2), qui est tourné vers le piston (3) de la seringue, possède un diamètre plus faible, qui permet sa libre mobilité.

5. Seringue selon l'une des revendications 1 à 4, caractérisée en ce que sur l'élément de fermeture (4) est emmanché un capuchon de sécurité (20), qui enserre selon une liaison par formes complémentaires l'élément de fermeture (4), le capuchon de sécurité (20) portant, sur son côté tourné vers l'appui-doigt (6), une bague de serrage (2.1) qui est raccordée, par l'intermédiaire d'une barrette de séparation mince, au capuchon de sécurité (20) et, à l'état complètement emmanché, est raccordée de façon inamovible à l'élément de fermeture (4), le capuchon de sécurité (20) portant sur son côté intérieur une tige axiale (20.2), qui ferme l'ouverture de la canule de l'élément de fermeture (4).
